(19) Europäisches Patentamt
European Patent Office
Office européen des brevets

(11) Veröffentlichungsnummer: **0 341 542 A2**

(12) # EUROPÄISCHE PATENTANMELDUNG

(21) Anmeldenummer: **89107889.1**

(22) Anmeldetag: **29.04.89**

(51) Int. Cl.4: **C12P 7/40 , C12P 41/00 , C12N 1/20 , //(C12P41/00, C12R1:07,1:39,1:39,1:64)**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4446-4447-4448-4453-4464-4465-4466

(30) Priorität: **07.05.88 DE 3815722**

(43) Veröffentlichungstag der Anmeldung: **15.11.89 Patentblatt 89/46**

(84) Benannte Vertragsstaaten: **AT DE ES FR GB IT NL SE**

(71) Anmelder: **BASF Aktiengesellschaft**
**Carl-Bosch-Strasse 38**
**D-6700 Ludwigshafen(DE)**

(72) Erfinder: **Ladner, Wolfgang, Dr.**
**In den Bellen 5**
**D-6701 Fussgoenheim(DE)**
Erfinder: **Siegel, Hardo, Dr.**
**Hans-Purrmann-Allee 25**
**D-6720 Speyer(DE)**

(54) **Verfahren zur Herstellung von Enantiomeren.**

**Es wird die selektive Hydrolyse von Verbindungen der Formel**

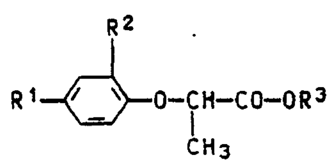

I,

**worin $R^1$ bis $R^3$ die in der Beschreibung genannte Bedeutung haben, beschrieben.**

EP 0 341 542 A2

## Verfahren zur Herstellung von Enantiomeren

(57) Eine Reihe von Phenoxypropionsäuren sowie deren Salze und Ester sind seit langem als Pflanzenschutzmittel bekannt, wie 2-Methyl-4-chlorphenoxypropionsäure oder 2,4-Dichlorphenoxypropionsäure. Von diesen Verbindungen besitzen nur die D-Enantiomeren biologische Aktivität. Trotzdem werden in der Praxis die Racemate dieser Verbindungen eingesetzt, da die Enantiomeren nur schwer und aufwendig herstellbar sind (EP 9285, DE 1 543 841). Es sind auch bereits Verfahren zur Herstellung enantiomerenreiner Phenoxypropionsäuren beschrieben, die auf eine enzymatische Racematspaltung der entsprechenden racemischen Ester beruhen (Biotechnol. Bioeng. 26, 1449 (1984), DE 3 532 026, Derwent Abstr. 86-202283/31). Diese Verfahren haben jedoch den Nachteil, daß sie sich auf wichtige Herbizid-wirksame Substanzen nicht anwenden lassen oder nur schlechte Ausbeuten liefern.

Gegenstand der Erfindung ist ein Verfahren zur selektiven Hydrolyse von Verbindungen der Formel I

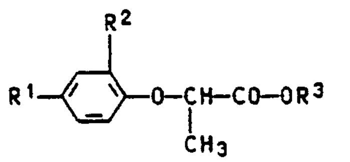

I,

worin
$R^1$ ein Wasserstoff- oder Halogenatom
$R^2$ ein Wasserstoff- oder Chloratom oder eine Methylgruppe und
$R^3$ eine $C_{1-5}$-Alkylgruppe
bedeuten, dadurch gekennzeichnet, daß man das Racemat der Verbindung der Formel I partiell enzymatisch verseift.

In der Formel I bedeutet $R^3$ vorzugsweise eine $C_{1-8}$-Alkylgruppe.

Unter partieller Verseifung ist die Spaltung lediglich eines Enantiomeren in dem Racemat zu verstehen. Dadurch entsteht aus dem racemischen Ester ein Gemisch aus D-Ester und L-Säure bzw. L-Ester und D-Säure.

Die partielle Hydrolyse wird mit Hydrolasen durchgeführt. Diese müssen nicht in reiner Form eingesetzt werden, vielmehr können auch ganze Zellen bzw. Mikroorganismen, die Hydrolasen enthalten, sowie Zell- bzw. Fermentationsüberstände mit Hydrolaseaktivität bzw. Zellaufschlüsse und Extrakte daraus verwendet werden.

Als Mikroorganismen sind insbesondere Bakterien der Gattungen Bacillus (vorzugsweise Bacillus sphäricus), Pseudomonas (vorzugsweise Pseudomonas fluorescens) und Xanthomonas zu nennen. Insbesondere sind zu nennen: DSM 4446-4448, 4464-4466 und 4453.

Die Hydrolasen können direkt oder in immobilisierter Form verwendet werden. Sollen ganze, Hydrolasen enthaltende Zellen immobilisiert werden, so gelingt das am einfachsten mit Hilfe von Calciumalginat. Lösliche Hydrolasen können beispielsweise durch kovalente Fixierung an ®Eupergit C (Oxirane acrylic beads erhätlich von Röhm Pharma GmbH) gebunden werden.

Die partielle Verseifung der Ester gelingt besonders gut bei 0 bis 50 °C, vorzugsweise 20 bis 30 °C, bei einem pH-Wert von 4 bis 9, vorzugsweise 5 bis 8. Da bei der Verseifung Säure entsteht, muß diese mit einer Base, wie Natronlauge, Kalilauge, Ammoniak oder aliphatischen niedermolekularen Aminen neutralisiert werden.

Die racemischen Ester können in der Reaktionsmischung in Konzentrationen von 0,1 bis 20 %, vorzugsweise von 1 bis 10 % vorliegen.

Das bei der partiellen Verseifung anfallende Gemisch an optisch aktivem Carbonsäuresalz und optisch aktivem Ester läßt sich aufgrund der unterschiedlichen Löslichkeiten in Wasser und organischen Lösungsmitteln leicht trennen.

Zur Hydrolyse der D-Enantiomeren eignen sich besonders:
Pronase E aus Streptomyces griseus (Sigma Chemie GmbH)
Protease Type I aus Pankreas, No. P 4630 (Sigma Chemie GmbH)
Pankreatin aus Schweinepankreas (Sigma Chemie GmbH)
sowie die Stämme DSM 4465, DSM 4466 und DSM 4453.

Die L-Carbonsäureester lassen sich mit folgenden Hydrolasen bzw. Mikroorganismen selektiv hydrolysieren:

Proteinase K aus Fungus No. P 0390 (Sigma Chemie GmbH)
Proteinase aus Bacillus subtilis (Fluka)
Piccantase A (Gist-Brocades)
DSM 4446 Bacillus sphäricus
DSM 4447 Bacillus sphäricus
DSM 4448 Bacillus sphäricus
DSM 4464 Pseudomonas fluorescens
Durch das erfindungsgemäße Verfahren werden die Verbindungen der Formel I erstmalig in großer Reinheit mit einfachen Mitteln zugänglich.

Isolierung der Mikroorganismen

Zu je 100 mg willkürlich gesammelten Bodenproben wurden 5 ml 0,9 %ige NaCl-Lösung gegeben und für 3 h bei 30 °C geschüttelt. 50 µl Proben davon wurden auf Agarplatten mit folgender Zusammensetzung ausplattiert:
4 g/l Bacto Nutrient Broth (Difco)
10 g/l Bacto Yeast Extrakt ( " )
5 g/l Glukose
20 g/l Agar
2 g/l Phenoxypropionsäurebutylester
0,2 g/l ®Tween 80
pH 7.0
Nach zweitägiger Inkubation bei 30 °C wurden die verschieden aussehenden Klone durch mehrmaliges Passagieren auf den gleichen Agarplatten gereinigt.

Die gereinigten Isolate wurden in 80 ml Schüttelkulturen auf Hydrolyse und Selektivität getestet. Die selektivsten Stämme waren die oben genannten DSM-Stämme.

Beispiel 1

2,0 g (8,0 mmol) racemischer Ester I ($R^1 = R^2 = Cl$, $R^3 = CH_3$) und 0,2 ml ®Tween 80 wurden in 50 ml 50 mM Tris/HCl-Puffer, pH 7,5, suspendiert und intensiv gerührt. Die Hydrolyse wurde durch Zugabe von 1 g Pronase (aus Streptomyces griseus, Fa. Sigma, Nr. P5147) gestartet. Während der Hydrolyse wurde der pH-Wert durch automatische Zudosierung von NaOH konstant gehalten. Nachdem 8 ml 0,5 N NaOH verbraucht worden waren, wurde die Reaktionsmischung mit HCl auf pH 1,5 eingestellt und zweimal mit 100 ml $CH_2Cl_2$ extrahiert. Zur Abtrennung der Säure wurde die organische Phase mit 30 ml 10 %iger $NaHCO_3$-Lösung extrahiert. Trocknen der organischen Phase mit $Na_2SO_4$ und Abdampfen des Lösungsmittels ergaben 0,8 g Ester mit einer Enantiomerenreinheit von ≧ 98 %.

Die Säure wurde aus der wäßrigen Phase nach Ansäuern mit HCl durch Extraktion mit $CH_2Cl_2$, Trocknen und Abdampfen des Lösungsmittels gewonnen. Ausbeute: 1,0 g, Enantiomerenreinheit: 91 % (nach saurer Veresterung mit Methanol per HPLC bestimmt).

Analog Beispiel 1 wurden folgende Isomeren erhalten:

| Beispiel Nr. | $R^1$ | $R^2$ | $R^3$ | Auswaage | Enantiomerenreinheit |
|---|---|---|---|---|---|
| 2a | Cl | $CH_3$ | $CH_3$ | 0,94 g | 89 % |
| 2b | Cl | $CH_3$ | H | 0,92 g | 85 % |
| 3 | H | H | $n\text{-}C_4H_9$ | | 56 % |
| 4 | H | $CH_3$ | $CH_3$ | | 78 % |
| 5 | H | $CH_3$ | $n\text{-}C_4H_9$ | | 77 % |

Beispiel 6

In einem 250 ml Erlenmeyerkolben wurden 80 ml steriles FPGH-Nährmedium (4 g/l Bacto Nutrient

Broth (Difco), 20 g/l Bacto Yeast Extrakt (Difco), 10 g/l Glukose, pH 7.0) mit DSM 4465 angeimpft und 20 h bei 30 °C unter Schütteln (200 rpm) inkubiert. Zu dieser Zellsuspension gab man 800 mg 2-Methyl-4-chlorpropionsäurebutylester und 40 mg ®Tween 80 und inkubierte 3 weitere Tage bei 30 °C. Man arbeitete wie in Beispiel 1 auf. Es wurden 380 mg Ester und 290 mg freigesetzte Säure isoliert. Das d/l-Verhältnis des nichthydrolysierten Esters wurde zu 18/82 bestimmt. Die freigesetzte Säure wies ein Enantiomerenverhältnis von d/l = 96/4 auf.

Beispiel 7

Analog Beispiel 6 wurde DSM 4466 gezüchtet und mit 2-Methyl-4-chlorpropionsäurebutylester inkubiert. Nach 3 Tagen waren laut GC 50 % des Esters hydrolysiert worden. Die d/l-Verhältnisse der freigesetzten Säure sowie des zurückgebliebenen Esters betrugen 96/4 bzw. 4/96.

## Ansprüche

1. Verfahren zur selektiven Hydrolyse von Verbindungen der Formel I

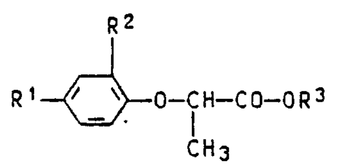

I,

worin
$R^1$ ein Wasserstoff- oder Halogenatom
$R^2$ ein Wasserstoff- oder Chloratom oder eine Methylgruppe und
$R^3$ eine $C_{1-5}$-Alkylgruppe
bedeuten, dadurch gekennzeichnet, daß man das Racemat der Verbindung der Formel I partiell enzymatisch verseift.

2. Mikroorganismen, ausgewählt aus der Gruppe bestehend aus DSM 4446 bis 4448, 4464-4466 und 4453.

3. Verwendung von Mikroorganismen, ausgewählt aus der Gruppe bestehend aus DSM 4446-4448, 4464-4466 und 4453, zur selektiven Hydrolyse von Verbindungen der Formel I gemäß Anspruch 1.